# EUROPÄISCHE PATENTSCHRIFT

(11) **EP 1 363 680 B1**
(45) Veröffentlichungstag und Bekanntmachung des Hinweises auf die Patenterteilung: **20.10.2004**
(21) Anmeldenummer: 02710854.7
(22) Anmeldetag: 15.02.2002
(51) Int. Cl.: A61L 15/58, A61L 15/60, A61F 13/15

(54) **TROCKEN-WIRBELBETTVERFAHREN ZUR HERSTELLUNG DÜNNER ABSORBIERENDER FLÄCHENGEBILDE**
METHOD USING A DRY FLUIDISED BED FOR PRODUCING THIN, ABSORBENT SURFACE STRUCTURES
PROCEDE EN LIT FLUIDISE DE TYPE SEC POUR LA PRODUCTION DE STRUCTURES DE SURFACE MINCES ET ABSORBANTES

(30) Priorität: 19.02.2001 DE 10107667
(43) Veröffentlichungstag der Anmeldung: 26.11.2003
(73) Patentinhaber: BASF AKTIENGESELLSCHAFT, 67056 Ludwigshafen (DE)
(72) Erfinder: HEIDER, Wolfgang, 67434 Neustadt (DE); HOFMANN, Jürgen, 67069 Ludwigshafen (DE); WANIOR, Mariola, 63526 Erlensee (DE); FUNK, Rüdiger, 65527 Niedernhausen (DE); LICHT, Ulrike, 68309 Mannheim (DE); EHLE, Michael, 67071 Ludwigshafen (DE)
(74) Vertreter: Isenbruck, Günter, Dr.
(86) Internationale Anmeldenummer: PCT/EP2002/001660
(87) Internationale Veröffentlichungsnummer: WO 2002/066088

(56) Entgegenhaltungen:
- EP-A- 0 816 383
- US-A- 4 392 908
- US-A- 5 436 066
- US-A- 5 549 854
- US-A- 6 066 759

## Beschreibung

Die Erfindung betrifft ein Verfahren zur Herstellung von dünnen absorbierenden Flächengebilden, die in Hygieneprodukten wie Windeln, Inkontinenzprodukten, Wundauflagen oder Damenbinden zur Aufnahme von Körperflüssigkeiten Verwendung finden können.

Als Hygieneprodukt oder Hygieneartikel bezeichnet man hauptsächlich Erzeugnisse wie Windeln, Damenbinden, Inkontinenzprodukte oder Wundauflagen. Je nach Verwendungszweck eignen sich diese Hygieneprodukte zum Aufsaugen und Speichern von Körperflüssigkeiten wie Blut, Urin oder Wundflüssigkeit. Während früher die Hygieneprodukte überwiegend reine Cellulose als saugaktives Material beinhalteten, sind moderne Hygieneprodukte zumeist mehrschichtig, wobei den einzelnen Schichten besondere Aufgaben zugesprochen werden. Es wird unterschieden zwischen a) Schichten, die dem Körper zugewandt sind, b) Schichten, die der Kleidung zugewandt sind bzw. die auf der zu den zuvor genannten Schichten entgegengesetzten Seite des jeweiligen Hygieneproduktes liegen und c) Schichten, die sich im Inneren des Hygieneproduktes befinden.

Schichten, die sich auf der dem Körper zugewandten Seite des Hygieneproduktes befinden, haben zum einen die Aufgabe, den Transport der Körperflüssigkeiten in das Innere des jeweiligen Hygieneproduktes zu gewährleisten. Andererseits sollen sie auch die Rücknässung unterbinden, das heißt, die sich im Inneren des entsprechenden Hygieneprodukts befindliche Körperflüssigkeiten sollen nicht wieder zurück an die Haut gelangen. Schichten, die sich auf der Kleidung zugewandten Seite des Hygieneproduktes befinden, stellen hingegen in beiden Richtungen eine undurchdringliche Barriere für die Körperflüssigkeiten dar. Ihre Hauptaufgabe ist folglich in der Unterbindung der Anschmutzung von Kleidungsstücken oder sonstigen Materialen zu sehen, die mit dem jeweiligen Hygieneprodukt in Berührung stehen.

Der wichtigste Bestandteil eines jeden Hygieneproduktes sind absorbierende Flächengebilde, die sich (in der Regel) im Inneren des jeweiligen Hygieneproduktes befinden und auch als Absorptionsschichten oder innere Schichten bezeichnet werden. Die absorbierenden Flächengebilde dienen zur Aufnahme und zum Speichern der Körperflüssigkeiten und enthalten als Hauptbestandteile ein Trägermaterial und einen quellfähigen Stoff. Das Trägermaterial, welches zur Verteilung der Körperflüssigkeiten dient, ist in der Regel aus zwei oder mehreren (Träger-)Schichten aufgebaut, die miteinander verklebt sind. Der quellfähige Stoff, der zur Aufnahme bzw. Speicherung der Körperflüssigkeiten verwendet wird, ist in das Trägermaterial eingearbeitet, er wird auch als Absorptionsmittel bzw. Superabsorber bezeichnet.

Neben dem für jeden Stoff individuellen Quellvermögen hängt die Flüssigkeitsaufnahmekapazität eines jeden Hygieneproduktes auch von der Menge des eingearbeiteten. Absorptionsmittels ab. Allerdings ist dabei zu beachten, dass nur eine begrenzte Menge an Absorptionsmittel in die jeweiligen Hygieneprodukte eingearbeitet werden kann. Neben der Stabilität des Gesamtproduktes ist hierfür vor allem im Falle von Windeln oder Damenbinden ein mit zunehmender Dicke des Produktes abnehmender Tragekomfort ein Kriterium. Zudem gilt insbesondere für größere Produktstückzahlen, dass je dünner das Produkt ist, umso einfacher ist es zu lagern bzw. zu transportieren. Dabei ist zu berücksichtigen, dass hauptsächlich die inneren Schichten, das heißt diejenigen, die das Absorptionsmittel beinhalten, zur Gesamtdicke des Hygieneproduktes beitragen. Deswegen ist man bestrebt, möglichst dünne und somit auch leichtere Hygieneprodukte bzw. absorbierende Flächengebilde herzustellen, die trotzdem über eine hohe Flüssigkeitsaufnahmekapazität verfügen.

Das Hauptproblem bei der Herstellung dünner absorbierender Flächengebilde und folglich auch von insgesamt dünnen Hygieneprodukten ist die stabile Fixierung einer möglichst großen Menge von Absorptionsmittel an bzw. auf dem Trägermaterial. Das Absorptionsmittel wird beispielsweise mit Hilfe eines Klebstoffes auf einer ersten Trägerschicht fixiert. Das auf der ersten Trägerschicht fixierte Absorptionsmittel wird wiederum von einer zweiten, üblicherweise identischen Trägerschicht abgedeckt. Somit liegt ein sandwichartiges Flächengebilde vor, in welchem sich eine Absorptionsmittelschicht zwischen zwei Trägerschichten befindet. Wird beim Fixieren des Absorptionsmittels auf bzw. zwischen den Trägerschichten zuviel Klebstoff verwendet, lässt sich das Absorptionsmittel zwar stabil fixieren, aber es kann weder allzu dicht noch gleichmäßig auf dem Träger gepackt werden. Dadurch entstehen zwangsläufig dickere absorbierende Flächengebilde, zudem sind Klebstoffe in aller Regel auch teuer. Wird andererseits zu wenig Klebstoff verwendet, ist das Absorptionsmittel nicht stabil auf dem Träger fixiert, zudem sind die einzelnen Trägerschichten nur unzureichend miteinander verklebt. Dies hat zur Folge, dass (teilweise) Absorptionsmittel aus dem absorbierenden Flächengebilde herausbröselt und dieses aufgrund zu geringer Belegung an Absorptionsmittel unbrauchbar oder zumindest in seiner Absorptionsfähigkeit erheblich eingeschränkt ist. Für die Qualität eines absorbierenden Flächengebildes ist auch von Bedeutung, wie und in welcher Reihenfolge die einzelnen Komponenten miteinander verknüpft werden.

US-A-5 436 066 offenbart ein Wirbelbettverfahren, bei dem ein Hydrogel-formendes polymer material, Mikrofasern, ein Adhäsiv sowie ein Oberflächenbenetzer trocken verwirbelt werden, und das entstehende Material zwischen zwei Trägerschichten einer Windel eingebracht wird.

EP-A 0 033 235 beschreibt ein Verfahren zur Herstellung von Produkten zur Absorption von Körperflüssigkeiten. Hierbei werden quellbare Polymerpartikel mit einem Klebstoff gecoatet, um anschließend auf der Oberfläche eines wasserabsorbierenden Trägers fixiert zu werden. Das Coating der quellbaren Polymerpartikel mit dem Klebstoff kann auf unterschiedliche Weise erfolgen. Zum Beispiel können die Polymerpartikel mit der wässrigen Lösung eines Klebstoffes sorgfältig durchmischt werden. Nachteilig ist dabei, dass das daraus erhaltene Gemisch im Anschluss gründlich getrocknet werden muss, um das mit dem Kleber zugegebene Wasser wieder aus den quellbaren Polymerpartikeln zu entfernen. Andererseits können die quellbaren Polymerpartikel auch als Slurry in einem organischen Lösungsmittel vorliegen, worauf der Klebstoff in fester oder gelöster Form zugegeben wird. Dies erfordert ebenfalls ein gründliches Trocknen des erhaltenen Gemisches. Das Gleiche gilt, wenn der Klebstoff als Lösung auf die Oberfläche der quellbaren Polymerpartikel aufgesprüht wird. Alternativ kann der Klebstoff schon während des Herstellungsverfahrens der quellbaren Polymerpartikel in diese eingebaut werden. Letzteres Verfahren ist aber mit einem sehr großen synthetischen Aufwand verbunden.

Allen vorstehend beschriebenen Coating-Methoden haftet aber ein weiterer Nachteil an, weil das Gemisch aus quellbarem Polymer und Klebstoff vor dem Auftragen auf das Trägermaterial zusätzlich gemahlen werden muss, um kleinteilige Partikel in Pulverform zu erhalten, die auf dem Trägermaterial verteilt werden können. Auf diese Weise können absorbierende Flächengebilde hergestellt werden, die 100 g/m² quellbares Polymer bezogen auf die Oberfläche des Trägermaterials enthalten. Die absorbierenden Flächengebilde werden bei 160 bis 180°C gepresst.

GB-A 2 004 201 beschreibt eine weitere Möglichkeit, wie Absorptionsmittel auf einer Trägerschicht fixiert werden kann. Das Absorptionsmittel wird hierbei auf dem Träger weitflächig ausgebreitet und anschließend mit einer wässrigen Lösung eines Klebstoffes besprüht. Nachteilig bei dieser Methode ist, dass die Trägerschicht durch das Aufsprühen der wässrigen Klebstofflösung feucht wird. Aus diesem Grund muss die Trägerschicht eine beträchtliche Mindestdicke aufweisen, da feuchte Trägerschichten, insbesondere solche auf Cellulosebasis, sehr leicht reißen und folglich nicht weiterverarbeitet werden können.

Saugfähige Hygieneprodukte, die mindestens eine Schicht aus einem Textilmaterial und einem darin eingeschlossenen quellfähigen Naturstoff umfassen, werden in DE-A 43 43 947 offenbart. Quellfähige Polysaccharide werden über ein divalentes Vernetzungsmittel, vorzugsweise Glyoxal, unter Ausbildung von kovalenten Bindungen an den Träger angebunden. Alternativ werden auch formaldehydfreie Acrylatkleber auf den Träger aufgesprüht. Die hierbei erhaltenen Hygieneprodukte weisen allerdings im trockenen Zustand eine beträchtliche Gesamtdicke von 3 bis 7 mm auf

Die der Erfindung zugrunde liegende Aufgabe besteht in der Bereitstellung von absorbierenden Flächengebilden, bei denen das Absorptionsmittel zwischen den Schichten des Trägermaterials möglichst dicht gepackt und stabil fixiert ist. Gleichzeitig sollen die absorbierenden Flächengebilde in trockenem Zustand auch möglichst dünn sein.

Erfindungsgemäß wird diese Aufgabe durch ein Verfahren zur Herstellung von absorbierenden Flächengebilden gelöst, das folgende Schritte aufweist:
A) Aus einem als Dispersion vorliegenden Klebstoff und einem teilchenförmigen Absorptionsmittel wird durch Verwirbelung der Komponenten ein Gemisch erzeugt,
B) das Gemisch aus Klebstoff und Absorptionsmittel wird gleichmäßig auf die Oberfläche einer Seite eines ersten flächigen Trägermaterials aufgebracht,
C) eine Seite eines zweiten flächigen Trägermaterials wird auf das Gemisch aus Klebstoff und Absorptionsmittel, welches sich auf der Oberfläche einer Seite des ersten flächigen Trägermaterials befindet, aufgebracht, wodurch ein sandwichartiges Zwischenprodukt erhalten wird, und
D) das sandwichartige Zwischenprodukt wird bei einer Temperatur von mindestens 90°C, einem Druck von 4 bis 50 bar und einer Presszeit von 0,5 bis 5 min zum Endprodukt verarbeitet.

Der Vorteil der erfindungsgemäßen Lösung liegt insbesondere darin, dass aufgrund des verbesserten Verfahrens zum Aufbringen des Klebstoffes auf die Absorptionsmittelpartikel, diese sowohl untereinander unter Ausbildung einer zweidimensionalen Schicht vernetzt werden als auch gleichzeitig eine stabile Vernetzung zum Trägermaterial in der dritten Raumrichtung hergestellt wird. Demzufolge lassen sich die Absorptionsmittelpartikel wesentlich dichter zu stabilen Schichten packen, ohne dass die Gefahr des Herausbröselns von Absorptionsmittelpartikeln aus dem absorbierenden Flächengebilde besteht. Die stabile Vernetzung der Schicht aus Absorptionsmittelpartikeln mit den sich sowohl darüber wie darunter befindlichen Schichten des Trägermaterials bewirkt eine deutliche Verbesserung der Haftungseigenschaften der einzelnen Schichten untereinander. Aufgrund des sehr dicht gepackten Absorptionsmittels sind solche dünnen absorbierenden Flächengebilde auch sehr leistungsstark hinsichtlich ihrer. Absorptionsfähigkeit. Folglich können leistungsstarke absorbierende Flächengebilde hergestellt werden, die aber trotzdem nur eine Gesamtdicke von ca. 1 mm aufweisen.

Das Aufbringen einer Dispersion des Klebstoffes stellt einen weiteren Vorteil der erfindungsgemäßen Lösung dar. Da das verwendete Dispersionsmittel von den Absorptionsmittelpartikeln aufgesogen wird, ist im Gegensatz zu herkömmlichen Verfahren weder das Trocknen noch das Zermahlen des Gemisches aus Absorptionsmittelpartikeln und Klebstoff notwendig. Aufgrund der relativ geringen Menge an Dispersionsmittel, wird die Absorptionsfähigkeit des mit Klebstoff bedeckten Absorptionsmittels nur geringfügig im Vergleich zur Absorptionsfähigkeit des unbedeckten Absorptionsmittels geschmälert. Zudem wird gegenüber herkömmlichen Verfahren relativ wenig Klebstoff, bezogen auf die Menge an Absorptionsmittel, verwendet, wodurch die Produktionskosten gesenkt werden.

Ein weiterer Vorteil der erfindungsgemäßen Lösung liegt darin, dass die Dicke der (verwendeten) Schichten aus Trägermaterial erheblich reduziert werden kann. Während bei herkömmlichen Verklebungsverfahren die Trägermaterialschichten, die zumeist aus Cellulose(-derivaten) bestehen, durch den aufgebrachten Klebstoff und/oder das aufgebrachte Absorptionsmittel befeuchtet werden, müssen diese Schichten über eine gewisse Mindestdicke verfügen, da eine feuchte Trägerschicht umso leichter reißen kann, je dünner sie ist. Bei der erfindungsgemäßen Lösung wird hingegen das Gemisch aus Kleber und Absorptionsmittel im trockenen Zustand auf die Trägerschicht aufgebracht. Deshalb kann die Dicke dieser Trägerschicht beträchtlich bis auf Werte von ca. 0,1 mm reduziert werden, ohne dass Gefahr besteht, dass die Trägerschicht während des weiteren Verarbeitens reißt. Dies ist ein deutlicher Beitrag zur Reduzierung der Gesamtdicke des absorbierenden Flächengebildes.

Ein nicht zu unterschätzender weiterer Vorteil der erfindungsgemäßen Lösung macht sich bei der Lagerhaltung des Endproduktes bemerkbar. Hygieneprodukte werden in aller Regel nicht als Einzelstücke sondern in Packungen mit großer Stückzahl verkauft. Dünnere Hygieneprodukte bewirken somit deutlich reduzierte Kapazitäten für die Lagerhaltung, insbesondere beim Hersteller sowie beim Einzelhändler als auch für den Endverbraucher.

Ein zusätzlicher Vorteil der erfindungsgemäßen Lösung liegt in den verwendbaren Klebstoffen, die ein Pressen der absorbierenden Flächengebilde im Bereich von 100 bis 110°C ermöglichen, während in herkömmlichen Pressverfahren die absorbierenden Flächengebilde bei Temperaturen über 160°C gepresst werden müssen. Die erfindungsgemäße Lösung ist somit materialschonender und es lassen sich auch Energiekosten einsparen.

Der Schritt (A) des erfindungsgemäßen Verfahrens wird anhand einer Zeichnung nachstehend näher erläutert.

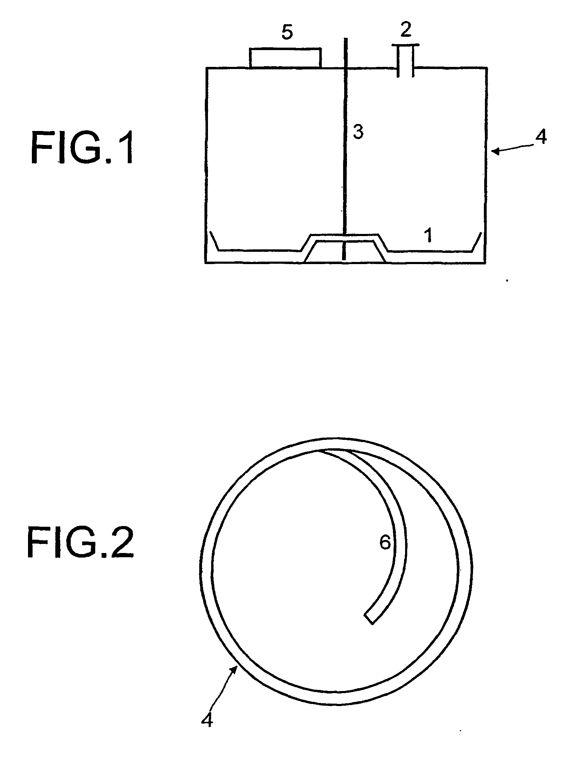

In Fig. 1 ist ein Querschnitt durch eine Vorrichtung zu sehen, in welcher ein Klebstoff auf ein Absorptionsmittel aufgebracht werden kann. Die Vorrichtung enthält im Wesentlichen ein Gehäuse 4, in dessen Inneren sich eine Drehachse 3 befindet, an die wiederum eine Rühreinrichtung 1 angebracht ist. Die Drehachse 3 ist vorzugsweise in der Mitte des Gehäuses 4 angebracht, die Rühreinrichtung 1 ist in einer bevorzugten Ausführung möglichst weit am unteren Ende der Drehachse 3 so angebracht, dass die Ausmaße der Rühreinrichtung 1 möglichst nahe an den Boden und/oder die Wand des Gehäuses 4 heranreichen. Als Rühreinrichtung 1 lassen sich alle dem Fachmann bekannten Vorrichtungen verwenden, die Drehachse 3 kann zudem über einen geeigneten Stellantrieb, bevorzugt einen elektrischen Stellantrieb, verfügen und gegebenenfalls von einer Recheneinheit gesteuert werden.

Das Gehäuse 4 ist bevorzugt zylinderförmig. Die Oberseite des Gehäuses 4 enthält einen abnehmbaren Deckel 5, der zum Einbringen sowie Herausnehmen von Substanzen in das bzw. aus dem Innere des Gehäuses 4 dient. Der Deckel 5 kann den üblichen Formen und Öffhungsgrößen beschaffen sein, gegebenenfalls kann sich der Deckel 5 auch über die gesamte Oberseite des Gehäuses 4 erstrecken. Ebenfalls an der Oberseite des Gehäuses 4 ist eine Einlassvorrichtung 2 (nachfolgend als Einlass 2 bezeichnet) angebracht, die zum Einbringen von Flüssigkeiten in das Innere des Gehäuses 4 verwendet werden kann. Als Einlass 2 eignen sich die dem Fachmann bekannten Vorrichtungen, insbesondere solche zum Einbringen von Dispersionen. Gegebenenfalls kann der Einlass 2 auch im oberen Drittel der Seite des Gehäuses 4 angebracht sein. In Fig. 2 ist zudem die Draufsicht auf die oben beschriebene Vorrichtung zu sehen. Daraus wird ersichtlich, dass im oberen Teil der Innenseite des Gehäuses 4 mindestens eine Vorrichtung 6 zum Umleiten von Partikelströmen (nachfolgend als Umleitungsvorrichtung 6 bezeichnet), insbesondere ein oder mehrere Leitbleche, angebracht sind. Die Dimensionen hinsichtlich der Größe, der Dicke und der geometrischen Formen der Umleitungsvorrichtung 6 sind dem Fachmann bekannt.

Das Absorptionsmittel wird durch Öffnen des Deckels 5 in das Innere des Gehäuses 4 eingebracht. Vorzugsweise liegt das Absorptionsmittel in Form von feinen, staubartigen Partikeln vor, es kann aber auch grobkörniger in Form von Pulver oder Granulat vorliegen. Insbesondere eignen sich Partikelgrößen zwischen 100 und 850 µm. Durch Rotieren der Drehachse 3 und der damit verbundenen Rühreinrichtung 1 wird das Absorptionsmittel im Inneren des Gehäuses 4 so aufgewirbelt, dass es sich über ein größtmögliches Raumvolumen verteilt und/oder keine Partikel des Absorptionsmittels auf dem Boden des Gehäuses 4 liegen bleiben. Die Umleitungsvorrichtung 6 dient hierbei zur Umleitung bzw. Beeinflussung der Partikelströme, insbesondere zu einer zusätzlichen Durchmischung der aufgewirbelten Partikel bzw. Partikelströme. Wenn das Absorptionsmittel sich im Inneren des Gehäuses 4 in aufgewirbeltem Zustand befindet, wird der Klebstoff durch den Einlass 2 zugegeben. Der Klebstoff liegt als Dispersion, bevorzugt als wässrige, emulgatorfreie Dispersion, vor. In einer bevorzugten Ausführung des erfindungsgemäßen Verfahrens wird die Dispersion des Klebstoffs auf die aufgewirbelten Absorptionsmittelpartikel aufgesprüht. Wesentlich ist hierbei, dass durch eine geeignete Ausführung der Einlass 2 die Dispersion des Klebstoffs möglichst fein verteilt über die aufgewirbelten Absorptionsmittelpartikel gesprüht wird.

Bevorzugt werden Dispersionen verwendet, die 20 bis 50 Gew.-% an Klebstoff enthalten, besonders bevorzugt 35 bis 45 Gew.-%. Die Menge an Dispersion, die durch den Einlass 2 zugegeben wird, richtet sich nach der Menge an Absorptionsmittel. Es werden 1 bis 20 Gew.-%, bevorzugt 1 bis 10 Gew.-%, besonders bevorzugt 4 bis 6 Gew.-% des Klebstoffes, bezogen auf das Gewicht des Absorptionsmittels, eingesetzt.

Im Anschluss an das Aufbringen des Klebestoffs auf die Absorptionsmittelpartikel wird die Rotationsbewegung der Rühreinrichtung 1 gestoppt und das Gemisch, welches aus dem Absorptionsmittel und dem Klebstoff besteht, kann, nachdem es sich gesetzt hat, aus dem Inneren des Gehäuses 4 direkt ohne weitere Trocknungs- und Zermahlschritte zur Weiterverarbeitung herausgenommen werden. Das Gemisch liegt bevorzugt in Form feiner Partikel vor.

Durch den zuvor beschriebenen Schritt (A) des erfindungsgemäßen Verfahrens wird eine möglichst großflächige Verteilung des Klebstoffs auf möglichst viele Partikel des Absorptionsmittels erzielt. Es ist dabei nicht zwingend beabsichtigt, dass alle Partikel des Absorptionsmittels vollständig mit Klebstoff überzogen werden. Bevorzugt ist vielmehr nur ein partielles Überziehen bzw. Anhaften des Klebstoffes auf den einzelnen Absorptionsmittelpartikel erwünscht, d.h., die Oberfläche der jeweiligen Partikel ist an einer oder mehreren Stellen mit Klebstoff überzogen. Dies kann durch die oben beschriebenen Gewichtsrelationen zwischen Klebstoff, Dispersionsmittel und Absorptionsmittel wirkungsvoll erzielt werden. Selbstverständlich ist aber durch Zugabe von größeren Mengen an Klebstoff auch ein größerflächiges bis vollständiges Überziehen der Absorptionsmittelpartikel mit Klebstoff möglich.

Weiterhin ist es auch möglich, anstelle eines Absorptionsmittels, Gemische aus zwei oder mehreren unterschiedlichen Arten von Absorptionsmitteln und /oder, anstelle eines Klebstoffes, Gemische aus zwei oder mehreren unterschiedlichen Arten von Klebstoffen in dem erfindungsgemäßen Verfahren einzusetzen.

Absorptionsmittel, die sich für das erfmdungsgemäße Verfahren zur Herstellung von dünnen absorbierenden Flächengebilden eignen, sind in WO 00/22018 beschrieben und unter Bezugnahme in die vorliegende Erfindung aufgenommen.

Als Absorptionsmittel eignen sich bevorzugt Polymere, die Struktureinheiten enthalten, die sich von (Meth-)Acrylsäure oder deren Estern ableiten, oder die durch Propf(co)polymerisation von (Meth-)Acrylsäure oder (Meth-)Acrylsäureester auf eine wasserlösliche Polymermatrix erhalten wurden. Als Pfropfgrundlage eignen sich beispielsweise Stärke, Cellulose, Cellulosederivate, Polyvinylalkohole, Polyalkylenoxide, Polyamine, Polyamide oder hydrophile Polyester. Weiterhin eignen sich bevorzugt Copolymere, die als Monomerenbausteine Stärke und Acrylnitril, Vinylacetat und Acrylester oder Acrylnitril und Acrylamid enthalten.

Bevorzugt liegen die Absorptionsmittel vernetzt vor, d. h. die oben beschriebenen (Co)polymere enthalten mindestens eine Einheit mit mindestens zwei Doppelbindungen, die in das Polymemetzwerk einpolymerisiert sind. Hierfür eignen sich insbesondere Methylenbisacrylamid, Methylenbismethacrylamid, Diacrylate oder Triacrylate.

Weiterhin liegen in den oben beschriebenen Absorptionsmitteln, die gegebenenfalls vernetzt sind, die gegebenenfalls vorhandenen säuregruppehaltigen Monomerenbausteine bevorzugt mit einem Neutralisationsgrad von 50 bis 85 Mol-% als Salz vor, insbesondere als Alkali- oder Ammoniumsalz.

Besonders bevorzugt eignen sich Absorptionsmittel, die mit Diacrylaten vernetzte Poly(meth)acrylsäure enthalten und deren säuregruppenhaltige Monomerenbausteine mit einem Neutralisationsgrad von 50 bis 85 Mol-% als Natriumsalz vorliegen.

Für das erfindungsgemäße Verfahren zur Herstellung von dünnen absorbierenden Flächengebilden eignen sich alle Klebstoffe, die unter den zuvor genannten Druck- und /oder Temperaturbereichen ihre Klebewirkung entfalten.

Geeignete Klebstoffe sind z.B. die in EP-A 0 738 750 beschriebenen und unter Bezugnahme in die vorliegende Erfindung aufgenommenen Polyurethane. Diese Polyurethane enthalten als Monomerenbausteine
a) mindestens ein mehrwertiges Isocyanat mit 4 bis 30 Kohlenstoffatomen,
b1) zu 10 bis 100 Mol-%, bezogen auf die Gesamtmenge an Diolen, mindestens ein Diol mit einem Molekulargewicht von 500 bis 5000, wobei wenigstens 80 Gew.-% der Diole (b1) einen Schmelzpunkt von 30 bis 100°C aufweisen und deren Schmelzenthalpie in diesem Temperaturintervall mindestens 50 J/g beträgt,
b2) zu 0 bis 90 Mol-%, bezogen auf die Gesamtmenge an Diolen, mindestens ein Diol mit einem Molekulargewicht von 60 bis 500 g/mol.

Bevorzugt eignen sich hierbei Polyesterpolyurethane, d.h. Polyurethane aus mindestens einen Polyester als Diolkomponente und mindestens einem Diisocyanat mit 6 bis 17 Kohlenstoffatomen.

Besonders bevorzugt eignen sich Polyesterpolyurethane enthaltend als Monomerenbausteine
a) Toluylendiisocyanat und/oder Hexamethylendüsocyanat,
b1) Polyester aus Adipinsäure und Butandiol-1,4.

Derartige Klebstoffe sind im Handel beispielsweise als Luphen® D200A (Hersteller: BASF AG, Ludwigshafen) erhältlich.

Bevorzugt beträgt das Verhältnis der Monomerenbausteine (a) zu (b) ca. 1:1 (in Mol-%), es können aber auch (deutliche) Überschüsse von (a) oder (b) eingesetzt werden.

Gegebenenfalls können die Polyurethane mit einem PUD-Salz (Amin, das eine Natriumcarboxylatgruppe enthält) umgesetzt werden. Weiterhin können gegebenenfalls wasseremulgierbare, polyfunktionelle Isocyanate den Copolymeren zugesetzt werden.

Bevorzugte Klebstoffe für das erfindungsgemäße Verfahren sind weiterhin Copolymere, die aus mindestens einer ungesättigten Carbonsäure und mindestens einem Olefin erhältlich sind.

Besonders bevorzugt eignen sich hierfür Copolymere, die Acrylsäure und Ethen als Monomerenbausteine enthalten.

Derartige Klebstoffe sind im Handel beispielsweise als Poligen® WE 3 oder Poligen® WE 4 (Hersteller: BASF AG, Ludwigshafen)erhältlich.

Die zuvor beschriebenen Klebstoffe, die im erfindungsgemäßen Verfahren Verwendung finden, können als Dispersion mit oder ohne Emulgator, insbesondere als wässrige, emulgatorfreie Dispersion, eingesetzt werden.

In Schritt (B) des erfindungsgemäßen Verfahrens wird das Gemisch aus Absorptionsmittel und Klebstoff auf die Oberfläche einer Seite eines flächigen Trägermaterials aufgebracht. Dabei wird das Gemisch gleichmäßig auf der Oberfläche der Seite des flächigen Trägermaterials verteilt, so dass die einzelnen Partikel des Gemisches möglichst dicht gepackt sind, und die dabei entstandene Schicht an Partikel des Gemisches, die sich auf der Oberfläche einer Seite des flächigen Trägermaterials erstreckt, möglichst dünn ist. Die einzelnen Partikel des Gemisches lassen sich problemlos dicht packen, wobei sehr hohe Belegungsdichten, bevorzugt zwischen 200 und 450 g/m², bezogen auf die Oberfläche einer Schicht des Trägermaterials, besonders bevorzugt zwischen 350 und 400 g/m², aufgetragen werden können. Gegebenenfalls können auch Belegungsdichten aufgetragen werden, die kleiner als 200 g/m² sind.

Ein wichtiger Vorteil des erfindungsgemäßen Verfahrens ist darin zu sehen, dass das Gemisch aus Klebstoff und Absorptionsmittel in trockenem Zustand auf das Trägermaterial aufgebracht wird, weshalb die Dicke der Schichten des Trägermaterials erheblich reduziert werden kann. Die Schichtdicke des Trägermaterials kann problemlos auf Werte von ≤ 0,2 mm, bevorzugt ≤ 0,1 mm, reduziert werden, ohne dass Gefahr besteht, dass die Trägerschicht während des weiteren Verarbeitens reißt. Gegebenfalls können auch problemlos Trägermaterialien mit Schichtdicken > 0,2 mm mit Gemischen aus Klebstoff und Absorptionsmittel belegt werden.

Im erfindungsgemäßen Verfahren eignet sich zum Herstellen von dünnen absorbierenden Flächengebilden als Trägermaterial insbesondere Cellulose. Weiterhin eignen sich Cellulosederivate, sonstige Polysaccharide sowie weitere dem Fachmann bekannte Trägermaterialien.

In Schritt (C) des erfindungsgemäßen Verfahrens wird eine Seite eines zweiten flächigen Trägermaterials auf die in Schritt (B) erzeugte Schicht aus Partikel des Gemisches aus Klebstoff und Absorptionsmittel aufgebracht, so dass letztere sich zwischen der ersten und der zweiten Trägermaterialschicht befindet. Die Anordnung aus der ersten Trägermaterialschicht, der Schicht aus Partikel des Gemisches aus Klebstoff und Absorptionsmittel und der zweiten Trägermaterialschicht kann als sandwichartiges Zwischenprodukt angesehen werden. Die beiden Trägerschichten können sich hinsichtlich ihrer Flächendimension, ihrer Dicke und/oder des verwendeten Materials unterscheiden. In einer bevorzugten Ausführung des erfindungsgemäßen Verfahrens sind das erste und die zweite flächige Trägermaterial jedoch identisch hinsichtlich ihrer Flächendimension, ihrer Dicke und des verwendeten Materials. Weiterhin können gegebenenfalls auch zwei oder mehrere (eventuell unterschiedliche) flächige Trägermaterialschichten, die auf geeignete Weise miteinander verklebt sind, unabhängig voneinander an eine und / oder beide Seiten der Schicht aus Partikel des Gemisches aus Klebstoff und Absorptionsmittel im sandwichartigen Zwischenprodukt anschließen. Ebenso können zwischen den jeweiligen Trägerschichten wiederum Schichten aus Partikel des Gemisches aus Klebstoff und Absorptionsmittel eingefügt werden.

In Schritt (D) des erfindungsgemäßen Verfahrens wird das in Schritt (C) erzeugte sandwichartige Zwischenprodukt zum Endprodukt gepresst. Hierfür wird eine Pressvorrichtung verwendet, wie sie dem Fachmann geläufig ist, wobei das Zwischenprodukt von der Pressvorrichtung bevorzugt auf 0,3 mm zusammengedrückt wird. Die Presszeit beträgt 0,5 bis 5 min, bevorzugt 2 bis 4 min, besonders bevorzugt 3 min. Gegebenenfalls kann die Presszeit auch länger als 5 min andauern. Die zum Pressen notwendige Temperatur soll mindestens 90°C betragen, bevorzugt wird bei 100 bis 110°C gepresst, gegebenenfalls kann die Temperatur auch über 110°C betragen. Um eine möglichst konstante Presstemperatur zu erzielen, wird die Pressvorrichtung vor dem eigentlichen Pressvorgang für eine geeignete Zeitspanne vorgeheizt. Als Pressdruck werden 4 bis 50 bar, bevorzugt 20 bis 30 bar, besonders bevorzugt 25 bar, verwendet. Gegebenenfalls kann der Pressdruck auch kleiner als 4 bar bzw. größer als 50 bar sein.

Aus dem sandwichartigen Zwischenprodukt wird somit in Schritt (D) des erfindungsgemäßen Verfahrens als Endprodukt ein absorbierendes Flächengebilde erzeugt, das im Wesentlichen aus Trägermaterial und Absorptionsmittel aufgebaut ist. Dieses absorbierende Flächengebilde zeichnet sich dadurch aus, dass es im trockenen, ungequollenen Zustand sehr dünn ist, bevorzugt 1 mm und weniger. Des Weiteren zeichnet sich dieses absorbierende Flächengebilde durch eine sehr hohe Belegungsdichte an Absorptionsmittel aus. Die Belegungsdichte ist bevorzugt zwischen 200 und 450 g/m² bezogen auf die Oberfläche einer Schicht des Trägermaterials, besonders bevorzugt zwischen 350 und 400 g/m². Gegebenenfalls lassen sich auch problemlos Belegungsdichten erzielen, die kleiner als 200 g/m² betragen.

Sowohl die hohe Belegungsdichte als auch die geringe Dicke des absorbierenden Flächengebildes lassen sich nur deshalb erzielen, weil der Klebstoff auf das Absorptionsmittel gemäß des oben beschriebenen Schritts (A) des erfindungsgemäßen Verfahrens aufgebracht worden ist. Durch das partielle Überziehen möglichst vieler Partikel des Absorptionsmittels haften diese Partikel sowohl untereinander als auch an der Oberfläche der beiden Schichten des Trägermaterials. Infolge der sehr guten Haftungseigenschaften der einzelnen Schichten untereinander können die sehr dünnen absorbierenden Flächengebilde (Endprodukt) realisiert werden. Zudem zeichnen sich die absorbierenden Flächengebilde auch durch eine sehr hohe Belegungsdichte (bis zu 400 g/m² und mehr) aus, weil die eingebrachten Absorptionsmittelpartikel aufgrund der guten Verklebung nicht mehr nach dem Pressen aus dem absorbierenden Flächengebilde herausbröseln können.

Die nach dem erfindungsgemäßen Verfahren hergestellten dünnen absorbierenden Flächengebilde finden als Bestandteil von Hygieneprodukten Verwendung. Insbesondere dienen sie in Hygieneprodukten wie Windeln, Inkontinenzprodukten, Wundauflagen oder Damenbinden zur Aufnahme von Körperflüssigkeiten. Als Körperflüssigkeiten kommen insbesondere Blut, Urin oder Wundflüssigkeit in Betracht. Die dünnen absorbierenden Flächengebilde können aber auch in beliebigen anderen, dem Fachmann bekannten Hygieneprodukten Verwendung finden.

Selbstverständlich kann das nach dem erfindungsgemäßen Verfahren hergestellte Gemisch aus Klebstoff und Absorptionsmittel auch in Trägermaterialen eingearbeitet werden, die so beschaffen sind, dass daraus zwangsläufig dicke absorbierende Flächengebilde erhalten werden. Dies gilt vor allem für die Einarbeitung in Fluffs, insbesondere in Fluffs aus Cellulose(-derivaten).

Die nachfolgenden Beispiele sollen die Erfindung näher erläutern.

### Beispiele 1-4:

### Herstellung von dünnen absorbierenden Flächengebilden

In Schritt (A) werden in der Vorrichtung 20 g des Absorptionsmittels vorgelegt. Durch Rotieren der Drehachse 3 und der damit verbundenen Rühreinrichtung 1 wird das Absorptionsmittel im Inneren des Gehäuses 4 so aufgewirbelt, dass keine Partikel des Absorptionsmittels auf dem Boden des Gehäuses 4 liegen bleiben. Wenn das Absorptionsmittel im Inneren des Gehäuses 4 in aufgewirbeltem Zustand ist, wird 1 ml der wässrigen, emulgatorfreien Dispersion des jeweiligen Klebstoffes über einen Zeitraum von 1 min durch die Einlass 2 in das Innere des Gehäuses 4 zugegeben. Anschließend wird die Rühreinrichtung 1 gestoppt und das Gemisch aus Absorptionsmittel und Klebstoff aus der Vorrichtung entfernt.

| | | | | |
|---|---|---|---|---|
| Beispiel Nr. | 1 | 2 | 3 | 4 |
| verwendeter Klebstoff | K1^{a} | K2^{b} | K2^{c} | K3 |
| Feststoffgehalt der Dispersion [%] | 40 | 24-26 | 20-22 | 40 |
| Die verwendeten Klebstoffe enthalten im Wesentlichen: K1: Polyesterpolyurethane, die als Monomerenbausteine Toluylendiisocyanat und Polyester aus Adipinsäure und Butandiol-1,4 enthalten, oder K2: Copolymere, die als Monomerenbausteine Acrylsäure und Ethen enthalten, oder K3: Polyesterpolyurethane, die als Monomerenbausteine Hexamethylendiisocyanat und Polyester aus Adipinsäure und Butandiol-1,4 enthalten. | | | | |

| | | | | |
|---|---|---|---|---|
| ^{a}: Der verwendete Klebstoff ist im Handel unter der Produktbezeichnung Luphen® D200A (Hersteller: BASF AG, Ludwigshafen) erhältlich. | | | | |
| ^{b}: Der verwendete Klebstoff ist im Handel unter der Produktbezeichnung Poligen® WE 3 (Hersteller: BASF AG, Ludwigshafen) erhältlich. | | | | |
| ^{c}: Der verwendete Klebstoff ist im Handel unter der Produktbezeichnung Poligen® WE 4 (Hersteller: BASF AG, Ludwigshafen) erhältlich. | | | | |

Das Absorptionsmittel enthält im Wesentlichen mit Diacrylaten vernetzte Polyacrylsäure, wobei die säuregruppenhaltige Monomerenbausteine mit einem Neutralisationsgrad von 50 bis 85 Mol-% als Natriumsalz vorliegen.

Schritt (B) wird direkt im Anschluss durchgeführt, wobei das zuvor erhaltene Gemisch aus Absorptionsmittel und Klebstoff gleichmäßig und als möglichst dünne Schicht auf eine Seite eines Cellulosetuches (Schichtdicke: ca. 0,1 mm; Länge: 200 mm; Breite: 200 mm) aufgetragen wird. Dabei lassen sich Belegungsdichten von ca. 400 g/m² bezogen auf die Oberfläche einer Seite des Cellulosetuches einstellen.

In Schritt (C) wird eine Seite eines zweiten, identischen Cellulosetuches auf die Oberfläche der dünnen Schicht des Gemisches aus Absorptionsmittel und Klebstoff aufgelegt, wodurch das sandwichartige Zwischenprodukt erhalten wird.

In Schritt (D) wird das sandwichartige Zwischenprodukt mit einer Laborpresse (Hersteller: IWK; Modell: HY 1086 (1956)) zum Endprodukt gepresst. Zwischen den beiden Pressflächen und den entsprechenden Seiten des Zwischenproduktes wird eine hitzebeständige Folie eingelegt. Nachdem die Presse für 3 Minuten vorgewärmt wurde, erfolgt das Pressen des Zwischenproduktes bei einer Temperatur von 100°C (Bsp. 1) bzw. 110°C (Bsp. 2-4), einem Druck von 25 bar sowie einer Dauer von 3 Minuten. Der Abstand zwischen den beiden Pressflächen wird mit einem Abstandsmesser auf 0,3 mm eingestellt.

Für alle 4 Beispiele (Bsp. 1-4) werden auf diese Weise stabile absorbierende Flächengebilde (Endprodukt) hergestellt, die im trockenen Zustand eine Dicke von 1 mm aufweisen und die stabil sowie flexibel sind. Insbesondere bleibt festzuhalten, dass das in Schritt (B) des erfindungsgemäßen Verfahrens auf das Cellulosetuch aufgebrachte Gemisch vollständig in das absorbierende Flächengebilde eingebunden ist. D. h., dass bei Handhabung bzw. Belastung unter Praxisbedingungen aus dem so hergestellten absorbierenden Flächengebilde keinerlei Absorptionsmittel bzw. Gemisch aus Klebstoff und Absorptionsmittel herausbröselt.

### (Vergleichs-)Beispiel 5:

### Herstellung von absorbierenden Flächengebilden unter Verwendung eines aufgeschäumten Klebstoffes

Der verwendete Klebstoff (im Handel unter der Produktbezeichnung Luphen® D200A (Hersteller: BASF AG, Ludwigshafen) erhältlich) enthält im Wesentlichen Polyesterpolyurethane, die als Monomerenbausteine Toluylendiisocyanat und Polyester aus Adipinsäure und Butandiol-1,4 enthalten. Der Klebstoff wird in Form eines Schaumes eingesetzt, der zu ca. 70 Gew.-% den Klebstoff enthält. Der Schaum wird auf jeweils eine Seite von zwei gleichgroßen Cellulosetüchem (Schichtdicke: ca. 0,5 mm; Länge: 200 mm; Breite: 200 mm) gleichmäßig aufgetragen, die beide anschließend mit dem in Beispiel 1 beschriebenen Absorptionsmittel belegt werden. Danach werden die beiden Cellulosetücher mit dem aufgebrachten Gemisch aus Klebstoff und Absorptionsmittel für 3 Minuten bei 100°C getrocknet. Nach dem Trocknen werden die beiden Cellulosetücher deckungsgleich so aufeinandergelegt, dass die beiden Klebstoff-Absorptionsmittel-Schichten vereinigt werden und ein sandwichartiges Zwischenprodukt erhalten wird. Die weitere Aufarbeitung des sandwichartigen Zwischenproduktes erfolgt analog zu Bsp.1, allerdings wird bei einem Druck von 50 bar für 5 Minuten gepresst.

Das daraus erhaltene absorbierende Flächengebilde ist gut verklebt und seine Schichtdicke beträgt im trockenen Zustand nach dem Pressen ca. 1 mm. Nachteilig ist allerdings, dass die Belegungsdichte an Absorptionsmittel (ca. 200 g/m² bezogen auf die Oberfläche einer Seite des Cellulosetuches) viel niedriger ist als in den Beispielen 1-4. Aufgrund des aufgeschäumten Klebstoffes kann die Oberfläche des Cellulosetuch weniger dicht mit Absorptionsmittel belegt werden. Zudem können im Gegensatz zu den Beispielen 1-4 keine Cellulosetücher mit einer Schichtdicke von ca. 0,1 mm verwendet werden, da diese durch Einwirkung des aufgeschäumten Klebstoffes sehr leicht reißen und somit unbrauchbar sind. Folglich kann generell weniger Absorptionsmittel in das so hergestellte absorbierende Flächengebilde eingearbeitet werden, um eine Gesamtdicke von ca. 1 mm nach dem Pressvorgang erzielen zu können. Weiterhin ist nachteilig, dass das so hergestellte absorbierende Flächengebilde im Gegensatz zu denjenigen der Beispiele 1-4 sehr steif und unflexibel ist.

### (Vergleichs-)Beispiel 6:

### Herstellung von absorbierenden Flächengebilden unter Verwendung eines pulverförmigen Klebstoffes

Als Klebstoff wird pulverförmiges, oxidiertes Polyethylenwachs (im Handel unter der Produktbezeichnung LUWAX® OA5 (Hersteller: BASF AG, Ludwigshafen) erhältlich) verwendet, welches zunächst mit dem in Beispiel 1 beschriebenen Absorptionsmittel durchmischt wird. Hierbei wird 10 Gew.-% Klebstoff bezogen auf die Menge an Absorptionsmittel eingesetzt. Das Gemisch aus Absorptionsmittel und Klebstoff wird möglichst gleichmäßig auf eine Seite eines Cellulosetuches (Schichtdicke: 1mm; Länge: 200 mm; Breite: 200 mm) aufgetragen und mit einer Seite eines zweiten, identischen Cellulosetuch abgedeckt. Die weitere Aufarbeitung erfolgt analog zu Beispiel 5.

Das auf diese Weise hergestellte absorbierende Flächengebilde genügt in keinerlei Hinsicht den Anforderungen. Die einzelnen Bestandteile des so hergestellten absorbierenden Flächengebildes sind sehr ungleichmäßig und unvollständig miteinander verklebt. Eine große Menge des eingebrachten Absorptionsmittels (anfängliche Belegungsdichte von ca. 400 g/m² bezogen auf die Oberfläche einer Seite des Cellulosetuches) bröselt bei Handhabung bzw. Belastung unter Praxisbedingungen aus dem absorbierenden Flächengebilde heraus. Die Belegungsdichte an fest eingebundenem Absorptionsmittel beträgt nur ca. 20% des ursprünglich aufgebrachten Wertes und ist somit viel niedriger als diejenigen in den Beispielen 1-4.

### Beispiele 7-9:

Anhand der nachfolgenden Beispiele 7-9 wird aufgezeigt, dass sich Absorptionsmittel (AM1+K), auf welches Klebstoff nach dem erfindungsgemäßen Verfahren aufgetragen wurde, hinsichtlich seiner Absorptionswirkung bzw. Quelleigenschaften gegenüber unbehandeltem Absorptionsmittel (u-AM1) nicht oder nur vernachlässigbar unterscheidet.

### Beispiel 7:

### Messung der Aufnahmekapazität (AK)

Die Aufnahmekapazität, gemessen in [g/g], gibt das maximale Absorptionsvermögen des jeweiligen Absorptionsmittel wieder.
0,2 g Absorptionsmittel werden in einen 60 x 85 mm großen Teebeutel eingewogen, der anschließend verschweißt wird. Der Teebeutel wird dann in einen Überschuss von 0,9 gew.-%iger Kochsalz-Lösung gegeben (mindestens 0,83 l Kochsalz-Lösung / lg Absorptionsmittel). Nach 60 Minuten Quellzeit wird der Teebeutel aus der Kochsalz-Lösung genommen, die überschüssige Kochsalz-Lösung abgetropft und der gequollene Teebeutel abgewogen. Die Aufnahmekapazität ergibt sich aus dem Quotienten des Gewichts des gequollenen zum trockenen Absorptionsmittel.

### Beispiel 8:

### Messung der Retention (Rot.)

Die Retention, gemessen in [g/g], gibt das Rückhaltevermögen des jeweiligen Absorptionsmittel unter Belastung an.

Die Versuchsdurchführung erfolgt analog zum Beispiel 7. Allerdings wird der Teebeutel im Anschluss an die 60 minutige Quellzeit bei einer Beschleunigung von 250 g drei Minuten lang zentrifugiert. Anschließend wird der Teebeutel gewogen. Die Retention ergibt sich aus dem Quotienten des Gewichts des gequollenen, zentrifugierten zum trockenen Absorptionsmittel.

### Beispiel 9:

### Absorption unter Druck (AuD)

Die Absorption unter Druck, gemessen in [g/g], gibt das Ausmaß des Quellvermögens des jeweiligen Absorptionsmittel gegen eine von außen einwirkende Kraft an. Die Versuchsdurchführung erfolgt analog zum Beispiel 7. Allerdings werden auf den Teebeutel zusätzlich Gewichte aufgelegt, die auf das jeweilige Absorptionsmittel einen Druck von 0,3 psi (0, 0207 bar) bzw. 0,7 psi (0,0483bar) ausüben.

### Auswertung der Beispiele 7-9:

| Beispiel Nr. | 7 (AK) | 8 (Rot.) | 9A(AuD/0,3psi) | 9B(AuD/0,7psi) |
|---|---|---|---|---|
| AM1+K | 42,8 | 26,9 | 27,8 | 21,1 |
| u-AM1 | 43,8 | 28,8 | 27,0 | 20,7 |

Das verwendete Absorptionsmittel (AM1) enthält im Wesentlichen mit Diacrylaten vernetzte Polyacrylsäure, wobei die säuregruppenhaltige Monomerenbausteine mit einem Neutralisationsgrad von 50 bis 85 Mol-% als Natriumsalz vorliegen.
Der verwendete Klebstoff (K/im Handel unter der Produktbezeichnung Luphen® D200A (Hersteller: BASF AG, Ludwigshafen) erhältlich) enthält im Wesentlichen Polyesterpolyurethane, die als Monomerenbausteine Toluylendiisocyanat und Polyester aus Adipinsäure und Butandiol-1,4 enthalten.

Die Beispiele 7-9 zeigen somit auf, dass die Einarbeitung des mit Klebstoff behafteten Absorptionsmittel in absorbierende Flächengebilde nach dem erfindungsgemäßen Verfahren zu keinen nennenswerten negativen Veränderungen hinsichtlich der Absorptionswirkung bzw. Quelleigenschaften des verwendeten Absorptionsmittels führt.

### Bezugzeichenliste

- 1: Rühreinrichtung
- 2: Einlassvorrichtung
- 3: Drehachse
- 4: Gehäuse
- 5: Deckel
- 6: Vorrichtung zum Umleiten von Partikelströmen

## Patentansprüche

1. Verfahren zur Herstellung von absorbierenden Flächengebilden, bei dem Klebstoff und Absorptionsmittel auf ein Trägermaterial aufgebracht werden, **dadurch gekennzeichnet, dass**
A) ein Gemisch aus einem als Dispersion vorliegenden Klebstoff und einem teilchenförmigen Absorptionsmittel durch Verwirbelung der Komponenten erzeugt wird,
B) das Gemisch aus Klebstoff und Absorptionsmittel gleichmäßig auf die Oberfläche einer Seite eines ersten flächigen Trägermaterials aufgebracht wird,
C) eine Seite eines zweiten flächigen Trägermaterials auf das Gemisch aus Klebstoff und Absorptionsmittel, welches sich auf der Oberfläche einer Seite des ersten flächigen Trägermaterials befindet, aufgebracht wird, wodurch ein sandwichartiges Zwischenprodukt erhalten wird, und
D) das sandwichartige Zwischenprodukt bei einer Temperatur von mindestens 90°C, einem Druck von 4 bis 50 bar und einer Presszeit von 0,5 bis 5 min zum Endprodukt verarbeitet wird.

2. Verfahren nach Anspruch 1, **dadurch gekennzeichnet, dass** der Klebstoff als wässrige, emulgatorfreie Dispersion vorliegt.

3. Verfahren nach Anspruch 1 oder 2, **dadurch gekennzeichnet, dass** 1 bis 20 Gew.-%, bevorzugt 1 bis 10 Gew.-%, besonders bevorzugt 4 bis 6 Gew.-%, an Klebstoff bezogen auf die Menge an Absorptionsmittel verwendet werden.

4. Verfahren nach einem der Ansprüche 1 bis 3, **dadurch gekennzeichnet, dass** das Absorptionsmittel mit Diacrylaten vernetzte Poly(meth)acrylsäure enthält, wobei die säuregruppenhaltigen Monomerenbausteine mit einem Neutralisationsgrad von 50 bis 85 Mol-% als Natriumsalz vorliegen.

5. Verfahren nach einem der Ansprüche 1 bis 4, **dadurch gekennzeichnet, dass** der Klebstoff im Wesentlichen enthält:
A) Polyesterpolyurethane enthaltend als Monomerenbausteine
a1) Toluylendüsocyanat und/oder Hexamethylendiisocyanat,
a2) Polyester aus Adipinsäure und Butandiol-1,4
oder
B) Copolymere, die als Monomerenbausteine Acrylsäure und Ethen enthalten.

6. Verfahren nach einem der Ansprüche 1 bis 5, **dadurch gekennzeichnet, dass** das Trägermaterial Cellulose ist.

7. Verfahren nach einem der Ansprüche 1 bis 6, **dadurch gekennzeichnet, dass** die Schichtdicke des Trägermaterials ≤ 0,2 mm, bevorzugt ≤ 0,1 mm, ist.

8. Verfahren nach einem der Ansprüche 1 bis 7, **dadurch gekennzeichnet, dass** das sandwichartige Zwischenprodukt bei Temperaturen von 100 bis 110°C gepresst wird.

9. Verfahren nach einem der Ansprüche 1 bis 8, **dadurch gekennzeichnet, dass** das sandwichartige Zwischenprodukt bevorzugt bei einem Druck von 20 bis 30 bar, besonders bevorzugt bei 24 bis 26 bar, gepresst wird.

10. Verfahren nach einem der Ansprüche 1 bis 9, **dadurch gekennzeichnet, dass** das sandwichartige Zwischenprodukt bevorzugt in einer Presszeit von 2 bis 4 min, besonders bevorzugt in 2,5 bis 3,5 min min, gepresst wird.

11. Verfahren nach einem der Ansprüche 1 bis 10, **dadurch gekennzeichnet, dass** das absorbierende Flächengebilde 200 bis 450 g/m², bevorzugt 350 bis 400 g/m², an Absorptionsmittel, bezogen auf die Oberfläche einer Seite des Trägermaterials, enthält

12. Verfahren nach einem der Ansprüche 1 bis 11, **dadurch gekennzeichnet, dass** das absorbierende Flächengebilde eine Dicke von ≤ 1 mm hät.

13. Verwendung von dünnen absorbierenden Flächengebilden hergestellt nach einem der Ansprüche 1 bis 12, als Bestandteil von Hygieneprodukten zur Aufnahme von Körperflüssigkeiten, insbesondere als Bestandteil von Windeln, Inkontinenzprodukten, Wundauflagen oder Damenbinden.

14. Hygieneprodukt zur Aufnahme von Körperflüssigkeiten, insbesondere eine Windel, ein Inkontinenzprodukt, eine Wundauflage oder eine Damenbinde, enthaltend mindestens ein dünnes absorbierendes Flächengebilde hergestellt nach einem der Ansprüche 1 bis 12.

## Claims

1. A process for producing absorbent planar structures, in which adhesive and absorbent are applied to a backing material, which comprises
A) producing a mixture of an adhesive in dispersion form and a particulate absorbent by fluidizing the components,
B) applying the mixture of adhesive and absorbent uniformly to the surface of one side of a first planar backing material,
C) one side of a second planar backing material is applied to the mixture of adhesive and absorbent that is present on the surface of one side of the first planar backing material, so producing a sandwichlike intermediate, and
D) the sandwichlike intermediate is processed to the end product at a temperature of at least 90°C, a pressure of from 4 to 50 bar, and a pressing time of from 0.5 to 5 min.

2. A process as claimed in claim 1, wherein the adhesive is in the form of an aqueous, emulsifier-free dispersion.

3. A process as claimed in claim 1 or 2, wherein from 1 to 20% by weight, preferably from 1 to 10% by weight, with particular preference from 4 to 6% by weight, of adhesive are used, based on the amount of absorbent.

4. A process as claimed in any of claims 1 to 3, wherein the absorbent comprises diacrylate-crosslinked poly(meth)acrylic acid, the monomer building blocks containing acid groups being in the sodium salt form with a degree of neutralization of from 50 to 85 mol%.

5. A process as claimed in any of claims 1 to 4, wherein the adhesive essentially comprises:
A) polyester polyurethanes containing as monomer building blocks
a1) tolylene diisocyanate and/or hexamethylene diisocyanate,
a2) polyesters of adipic acid and 1,4-butanediol
or
B) copolymers containing acrylic acid and ethene as monomer building blocks.

6. A process as claimed in any of claims 1 to 5, wherein the backing material is cellulose.

7. A process as claimed in any of claims 1 to 6, wherein the layer thickness of the backing material is ≤ 0.2 mm, preferably ≤ 0.1 mm.

8. A process as claimed in any of claims 1 to 7, wherein the sandwichlike intermediate is pressed at temperatures of 100 to 110°C.

9. A process as claimed in any of claims 1 to 8, wherein the sandwichlike intermediate is pressed preferably at a pressure of from 20 to 30 bar, with particular preference from 24 to 26 bar.

10. A process as claimed in any of claims 1 to 9, wherein the sandwichlike intermediate is pressed preferably in a pressing time of from 2 to 4 min, with particular preference in from 2.5 to 3.5 min.

11. A process as claimed in any of claims 1 to 10, wherein the absorbent planar structure contains from 200 to 450 g/m², preferably from 350 to 400 g/m², of absorbent, based on the surface area of one side of the backing material.

12. A process as claimed in any of claims 1 to 11, wherein the absorbent planar structure has a thickness of ≤ 1 mm.

13. The use of thin absorbent planar structures produced as claimed in any of claims 1 to 12 as components of hygiene products to absorb body fluids, especially as components of diapers, incontinence products, wound contact materials or sanitary napkins.

14. A hygiene product for absorbing body fluids, in particular a diaper, an incontinence product, a wound contact material or a sanitary napkin, comprising at least one thin absorbent planar structure produced as claimed in any of claims 1 to 12.

## Revendications

1. Procédé de production d'objets plans absorbants, dans lequel on dépose un adhésif et un agent absorbant sur une matière support, **caractérisé en ce que**
A) l'on produit un mélange composé d'un adhésif se présentant sous forme de dispersion et d'un agent absorbant sous forme de particules en faisant tourbillonner les composants,
B) l'on dépose de manière homogène le mélange composé de l'adhésif et de l'agent absorbant sur la surface d'un côté d'une première matière support plane,
C) l'on dépose un coté d'une deuxième matière support plane sur le mélange composé de l'adhésif et de l'agent absorbant qui se trouve sur la surface d'un côté d'une première matière support plane, lors de quoi on obtient un produit intermédiaire de type sandwich, et
D) l'on traite le produit intermédiaire de type sandwich à une température d'au moins 90°C, sous une pression de 4 bars à 50 bars et en un temps de compression de 0,5 min à 5 min pour former le produit fini.

2. Procédé selon la revendication 1, **caractérisé en ce que** l'adhésif se présente sous forme de dispersion aqueuse exempte d'émulsifiants.

3. Procédé selon la revendication 1 ou 2, **caractérisé en ce que** l'on utilise 1% à 20% en poids, de préférence 1% à 10% en poids, de manière plus particulièrement préférée 4% à 6% en poids d'adhésif par rapport à la quantité d'agent absorbant.

4. Procédé selon l'une quelconque des revendications 1 à 3, **caractérisé en ce que** l'agent absorbant contient de l'acide poly(méth)acrylique réticulé avec des diacrylates, les composants monomères contenant des groupes acide étant présents avec un degré de neutralisation de 50% à 85% en moles sous forme de sel de sodium.

5. Procédé selon l'une quelconque des revendications 1 à 4, **caractérisé en ce que** l'adhésif contient principalement :
A) des polyester-polyuréthannes contenant, en tant que composants monomères,
a1) du diisocyanate de toluylène et/ou du diisocyanate d'héxaméthylène,
a2) des polyesters d'acide adipique et de 1,4-butanediol
ou
B) des copolymères qui contiennent, en tant que composants monomères, de l'acide acrylique et de l'éthène.

6. Procédé selon l'une quelconque des revendications 1 à 5, **caractérisé en ce que** la matière support est la cellulose.

7. Procédé selon l'une quelconque des revendications 1 à 6, **caractérisé en ce que** l'épaisseur de couche de la matière support est inférieure ou égale à 0,2 mm, de préférence inférieure ou égale à 0,1 mm.

8. Procédé selon l'une quelconque des revendications 1 à 7, **caractérisé en ce que** le produit intermédiaire de type sandwich est comprimé à des températures de 100°C à 110°C.

9. Procédé selon l'une quelconque des revendications 1 à 8, **caractérisé en ce que** le produit intermédiaire de type sandwich est comprimé de préférence sous une pression de 20 bars à 30 bars, de manière plus particulièrement préférée de 24 bars à 26 bars.

10. Procédé selon l'une quelconque des revendications 1 à 9, **caractérisé en ce que** le produit intermédiaire de type sandwich est comprimé de préférence en un temps de compression de 2 min à 4 min, de manière plus particulièrement préférée de 2,5 min à 3,5 min.

11. Procédé selon l'une quelconque des revendications 1 à 10, **caractérisé en ce que** l'objet plan absorbant contient 200 g/m² à 450 g/m², de préférence 350 g/m² à 400 g/m² d'agent absorbant par rapport à la surface d'un côté de la matière support.

12. Procédé selon l'une quelconque des revendications 1 à 11, **caractérisé en ce que** l'objet plan absorbant a une épaisseur inférieure ou égale à 1 mm.

13. Utilisation d'objets plans absorbants minces, produits selon l'une quelconque des revendications 1 à 12, en tant que composant de produits hygiéniques afin d'absorber des liquides corporels, en particulier en tant que composant de couches, de produits d'incontinence, de pansements ou de serviettes hygiéniques.

14. Produit d'hygiène pour absorber des liquides corporels, en particulier couche, produit d'incontinence, pansement ou serviette hygiénique contenant au moins un objet plan absorbant mince produit selon l'une quelconque des revendications 1 à 12.
